# EUROPEAN PATENT SPECIFICATION

(11) **EP 4 126 145 B1**
(45) Date of publication and mention of the grant of the patent: **03.09.2025**
(21) Application number: 21720078.1
(22) Date of filing: 25.03.2021
(51) Int. Cl.: A61M 16/00, A61M 16/12, A61M 16/20

(54) **MODULAR VENTILATION SYSTEM FOR TIME CONTROLLED ADAPTIVE VENTILATION OF ONE OR MORE PATIENTS OR LUNGS**
MODULARES BEATMUNGSSYSTEM ZUR ZEITGESTEUERTEN ADAPTIVEN BEATMUNG EINES ODER MEHRERER PATIENTEN ODER LUNGEN
SYSTÈME DE VENTILATION MODULAIRE POUR VENTILATION ADAPTATIVE À COMMANDE TEMPORELLE D'UN OU PLUSIEURS PATIENTS OU POUMONS

(30) Priority: 25.03.2020 US 202062994285 P; 30.03.2020 US 202063002349 P
(43) Date of publication of application: 08.02.2023
(73) Proprietor: Habashi, Nader M., Annapolis, MD 21409 (US)
(72) Inventor: Habashi, Nader M., Annapolis, MD 21409 (US)
(74) Representative: IP Maison
(86) International application number: PCT/US2021/024258
(87) International publication number: WO 2021/195449

(56) References cited:
- US-A- 3 974 828
- US-A- 4 060 078
- US-B1- 6 474 334

## Description

### Cross-Reference to Related Applications and Claim to Priority

This application claims priority to Provisional Application Serial No. 62/994,285 filed March 25, 2020, and Provisional Application Serial No. 63/002,349 filed March 30, 2020.

### Field of the Technology

The present technology relates to ventilation apparatus and systems for ventilation. More specifically, the present technology relates to a ventilation apparatus comprising modular components to provide time controlled adaptive ventilation to one or more individuals. According to the present technology, the components of the apparatus are easy to manufacture and assemble, inexpensive, portable, and may be disposable or simple to sterilize.

### Background of the Technology

The earliest breathing machine was the Drinker respirator. It was invented in 1928 and was known as an "iron lung". These machines were used from the 1930s and 1950s by patients whose breathing muscles had been paralyzed by polio. They used negative pressure to help patients breathe while lying inside the iron lung's airtight chamber. A pump removed air inside the chamber, creating low air pressure around the patient's chest. This enabled the lungs to expand, causing the patient to breathe in. Other negative-pressure devices included the "cuirass", a shell-like device that was tightly strapped over the chest. A pump created a vacuum across the chest, forcing the patient to breathe in.

These types of respirators were replaced by ventilators in the 1950s. Ventilators use positive pressure, meaning they actively pumped air directly into the lungs through a face mask or a tube in the throat. Ventilators were developed during the World War II to supply oxygen to fighter pilots flying at high altitude. Hand-pumped and mechanical positive-pressure devices helped patients breathe during polio epidemics in the 1950s. During this time, the development of ventilators that actively pumped air directly into and out of the lungs was credited with rapidly dropping the mortality rates in patients with polio from more than 80 percent to about 40 percent "almost overnight". (see Slutsky, A. S., History of Mechanical Ventilation - From Vesalius to Ventilator-induced Lung Injury, Am J Respir Crit Care Med. (May 15, 2015) 191(10):1106-15).

Ventilators have since become essential tools within surgery as well as in intensive-care medicine. In fact, mechanical ventilation is one of the most common interventions implemented in the intensive care unit (ICU). More than half of the patients in the ICU are ventilated the first 24 hours after ICU admission, including individuals who have acute respiratory failure, compromised lung function, difficulty breathing, or failure to protect their airway (see Kirton, O., Mechanical Ventilation in the Intensive Care Unit, AAST.org (2011) General Information, Mechanical Ventilation).

Accordingly, ventilators play an important role in the care of patients with compromised lung function. The lung's primary function is to add oxygen and to remove carbon dioxide from the blood passing through the lung's blood vessels. Anytime the lungs are compromised, the patient's ability to exchange oxygen and carbon dioxide becomes difficult. The lungs can be compromised if a patient has COVID-19, pneumonia or any other disease or condition that affects the lung's function. Ventilators basically assist patient's lungs with this function.

Modern ventilators operate in a defined ventilatory mode, which is the process by which the mechanical ventilator determines, either partially or fully, when the mechanical breaths are to be provided to the patient, thus determining the breathing pattern of the patient during mechanical ventilation.

Early ventilators provided only machine-triggered volume-control inspiration. However, as technology advanced, pressure-targeted ventilators were developed, and these became the norm.

Airway pressure release ventilation (APRV) was described in 1987, as a mode that allows spontaneous breathing throughout the ventilation cycle. (See Downs JB, Stock MC. Airway pressure release ventilation: a new concept in ventilatory support. Crit Care Med. 1987;15:459-461). APRV is a time-cycled alternant between two levels of positive airway pressure, with the main time on the high level and a brief expiratory release to facilitate ventilation.

Bird, U.S. Patent No. 3,974,828 discloses a ventilator with an inhalation phase and an exhalation phase in its operative cycle having an inlet adapted to be connected to a supply of gas under pressure and first, second and third outlets. A servo controller is provided having an inlet and an outlet with control valve means movable between open and closed positions to control the flow of gas from the inlet to the outlet. A conduit system is provided for supplying gas from the first outlet to the patient adapter. An exhalation valve assembly is coupled to the patient adapter and is movable between open and closed positions. A conduit system is provided for supplying gas from the second outlet to the exhalation valve assembly to maintain the exhalation valve assembly in a closed position during the time the gas is being supplied from the outlet of the servo controller. A sensing mechanism is provided for sensing the pressure of the gas in the conduit system for supplying gas from the outlet of the servo controller to the patient adapter and for switching the servo controller from an open position to a closed position when a predetermined pressure is reached. A flow acceleration system is provided for augmenting the flow of gases through the first outlet when the pressure in the first outlet is of a certain predetermined pressure. A fail-safe apneustic plateau system is provided. In addition, an expiratory termination circuit is provided for commencing the inspiratory phase with high constant positive pressures existing in the inspiratory breathing circle.

Bird, U.S. Patent No. 4,060,078 discloses a ventilator having an inhalation phase and an exhalation phase in its operative cycle for use with a source of gas under pressure. A demand flow accelerator is responsive to the pressure of the gases in a breathing head assembly and provides additional gases to the breathing head assembly when the pressure of the gases in the breathing head assembly falls below a predetermined pressure. A sensor is also provided responsive to the pressure of the gases in the breathing head assembly for supplying gases to the breathing head assembly when the pressure of the gases in the breathing head assembly falls below a predetermined value to cause the patient to exhale against a substantially constant positive airway pressure. An additional sensor is also provided which is sensitive to the airway pressure being sensed for bleeding gases from the breathing circuit when pressure greater than a predetermined pressure are reached. Lock-out means is provided for locking out an inspiratory phase which exceeds a predetermined time. Starting means is provided for ensuring that the ventilator will be switched to an expiratory phase before an inspiratory phase is initiated.

Bird, U.S. Patent No. 5,007,420 discloses a ventilator for use with a source of gas under pressure for supplying such gas to the airway of a patient having an inlet adapted to be connected to the source of gas, and an outlet adapted to be connected to the airway of the patient. A pneumatic oscillator is connected to the inlet for supplying pulsatile gas in the form of successive small volumes of gas to the airway of the patient during a breath of the patient to cause diffusive ventilation of the airway to the patient. An exhalation valve assembly is connected to the patient airway for permitting the patient to exhale gases introduced into the patient airway.

Lemer, U.S. Patent 6,474,334 discloses a ventilation system including a manifold fluidly connectable at an inlet thereof to a pressurized source of a driving gas, the manifold having a plurality of branches, an electronically controlled valve fluidly connected to each branch downstream of the inlet of the manifold, a venturi-type ventilator fluidly connected to each of the branches downstream of the valves, each venturi-type ventilator being operative to force a driving gas into lungs of a patient, and a controller in electrical communication with the valves, the controller operating the valves in accordance with an operating cycle including: a) causing a driving gas to flow through one of the ventilators while substantially preventing flow of the driving gas through the other ventilators, b) causing the driving gas to flow to another one of the ventilators which previously had no driving gas flowing therethrough, while substantially preventing flow of the driving gas through the other ventilators including the ventilator which previously had the driving gas flowing therethrough, and c) sequentially repeating steps a) and b) until all the ventilators have had the driving gas flow therethrough.

McDaniel, et al., U.S. Patent No. 9,314,579 discloses a ventilator that is small, lightweight, and portable, yet capable of being quickly adapted to operate in a plurality of different modes and configurations to deliver a variety of therapies to a patent. A porting system having a plurality of sensors structured to monitor a number of parameters with respect to the flow of gas, and a number of porting blocks is used to reconfigure the ventilator so that it operates as a single-limb or dual limb ventilator. In the single-limb configuration, an active or passive exhaust assembly can be provided proximate to the patient. The ventilator is capable of operate in a volume or pressure support mode, even in a single-limb configuration. In addition, a power control mechanism controls the supply of power to the ventilator from an AC power source, a lead acid battery, an internal rechargeable battery pack, and a detachable battery pack.

Koch, et al., WO 2012/062 266 discloses a device and a method for ventilation with gas and/or with liquid, containing a ventilation tube with an attached pressure sensor, an inspiration pump, which is attached to the tube and is controlled by a motor controller, an expiration pump, which is attached to the tube and is controlled by the motor controller, an oxygenator unit, which is connected at the inlet side to the expiration pump, a main container, which is connected to the inspiration pump and of which the temperature is stabilized by a dedicated thermostat, an additional container for liquid, which additional container is arranged between the outlet of the oxygenator unit and the inlet of the main container, a balance for determining the ventilated amount of liquid of the subject to be ventilated, and a control unit, which switches valves of the overall circuit in order to perform the ventilation and is connected at least to motor controller and sensors. Between the outlet side of the main container and the inlet side of the oxygenator unit, a peristaltic pump connecting the two is arranged for transporting the respective fluid from the main container to the oxygenator via hose lines, wherein a valve is connected to an additional container for gas, and wherein the control unit is designed at least with a first function block for calculating ventilation parameters for the gas ventilation, for the total liquid ventilation and for the combination of the two fluid ventilations in a first function unit, with a second function block for the motor control in a second function unit, and with a third function block for data acquisition in a third function unit.

Modern ventilators are complex and expensive medical equipment. During a health crisis, such as the COVI-19 pandemic, many patients may not receive adequate medical care because of a shortage of ventilators. While ventilators do not directly cure disease, they can save lives by supporting lung function to allow patients to recuperate. Accordingly, there is a need for a means of ventilating multiple patients from a single gas source that can be controlled individually for each patient, in a simple, cost effective, portable, disposable, modular and/or easy to assemble manner, and optionally without access to a power source.

### Summary of the Invention

The present invention provides a modular ventilation system as defined in claim 1, a method of ventilating at least one extracorporeal or test lung as defined in claim 13, a kit for a modular ventilation system as defined in claim 14, and a modular ventilator apparatus comprising the components of the kit of claim 14 as defined in claim 15.

### Summary of the Disclosure

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

The present disclosure relates to a modular ventilation system that provides ventilation to one patient or multiple patients simultaneously such as in mass causality situations or pandemics. The ventilation system connects to a single gas source, such that multiple patients may share the single gas source, and comprises components of individual ventilator apparatus that provide ventilation to each individual patient. Each ventilation apparatus is made of components that are modular and can easily be assembled in multiple configurations.

The components of the system are preferably stand-alone components that are disposable and modular, such that the system has modular configurations.

The system may utilize different power sources or be entirely mechanical and require no electrical power to provide maximum flexibility for different situations, including emergency situations. According to a preferred embodiment, the power source comprises a compressed gas driven turbine or a source of electricity such as a battery or an alternating current from a wall outlet or generator or power outlet. The battery may be rechargeable, such as by wind, solar, nuclear, or other source of electricity.

The gas source for the ventilation system supplies pressurized gas, which may utilize different gas sources to provide maximum flexibility for emergency situations, such as for example, compressed gas, high flow generator, turbine, or centrifugal pump. The gas source generates flow pressure and determines gas concentrations. The system may use a venturi to entrain different gases or a gas blender to obtain a preferred gas concentration or mixture.

According to a preferred embodiment, the venturi introduces oxygen to produce a gas flow with less than 100% oxygen, preferably about 70% oxygen. Alternatively, the gas blender introduces oxygen to produce a gas flow that is up to 100% oxygen. Preferably, the inspiratory patient pressure is no more than 100 cm H₂0, and the manifold pressure is 100 cm H₂O times a number of patients or lungs connected to the system. According to a preferred embodiment, the system further comprises an alarm that sounds when any of these pressure thresholds are not met.

The ventilation system connects the gas flow through a circuit configured to accommodate one or more patients. With more than one patient, the main limb for pressurized gas functions as a manifold to provide separate gas supply to each patient.

The gas pressure in the system is controlled by various valves, including a flow-pressure regulator, an inspiratory pressure relief valve, a time cycled pressure release valve, an expiratory pressure relief valve, and a manifold pressure regulator valve. The time cycled pressure release valve is time controlled. The expiratory pressure relief valve depressurizes the exhalation limb and is indirectly time controlled by the time cycled pressure release valve. The manifold pressure regulator valve regulates pressure in the manifold and can bleed off excess pressure.

The system of the present disclosure may regulate the pressure, the pressure relief, and the timing of ventilation. Alternatively, the system may allow for control of pressure regulation, pressure relief, and the duration of different pressures. The system may provide a patient or a lung with continuous positive pressure (CPAP) or cyclic ventilation by adjusting the inspiratory pressure relief valve and / or the expiratory pressure relief valve. Alternatively, the system may ventilate a patient or lung in a pressure and time-controlled mode by adjusting the inspiratory pressure relief valve and the time cycled pressure release valve.

According to a preferred embodiment, the inspiratory pressure relief valve, the time cycled pressure release valve, and the expiratory pressure relief valve are relatively closer to the patient or lung as opposed to being closer to the gas source, for ease of adjusting individual patient or lung ventilation parameters, especially when the gas source is remote from the patient. According to a more preferred embodiment, the inspiratory pressure relief valve, the time cycled pressure release valve, and the expiratory pressure relief valve are part of a patient wearable, for example, part of a mask or tubing.

The system may include a graphic display of information about the ventilation status, as well as a controller to control and adjust ventilation. A user interface can be placed at a distance away from the patient in the event of a highly contagious process. Data output may provide data streaming to remote location(s) as a central station for expert guidelines, clinical support, technical support, and device support.

An object of the present disclosure is to provide a modular ventilation system to ventilate at least one patient or lung, wherein the system comprises:
a. A port for connecting a single gas source to the system to create a gas flow,
b. A pressure-flow regulator to regulate a pressure in the gas flow,
c. At least one of a venturi or gas blender to introduce oxygen into the gas flow,
d. At least one system manifold to provide gas flow to the at least one patient or lung,
e. At least three pressure relief valves,
f. A time cycled pressure release valve, and
g. A time controller.

wherein the at least three pressure relief valves comprise an inspiratory pressure relief valve to regulate inspiratory patient pressure, an expiratory pressure relief valve to regulate expiratory patient pressure, and a manifold pressure regulator valve to regulate manifold pressure,
wherein the at least one system manifold comprises at least three connectors, comprising a source connector that connects a first manifold to the gas source, a patient connector that connects the at least one system manifold to a ventilator circuit that delivers the gas flow to the at least one patient or lung, and a manifold connector that connects the first manifold to the manifold pressure regulator valve or a second manifold, and
wherein the inspiratory pressure relief valve, the time cycled pressure release valve and the expiratory pressure relief valve are proximal to the patient or the lung, and individually adjustable for each patient or lung.

Preferably, the connectors are mechanical connections comprising threaded, snap, friction or quick connect connectors. More preferably, the pressure relief valves are mechanical valves comprising spring loaded pressure valves or pneumatic valves, or electrical valves comprising solenoid valves.

It is another object of the present disclosure to provide a method of ventilating at least one patient or lung with the above-mentioned modular ventilation system.

It is yet another object of the present disclosure to provide a kit for a modular ventilation system comprising:
a. At least two stand-alone system manifolds each comprising three manifold connectors,
b. A stand-alone pressure-flow regulator,
c. At least two stand-alone ventilator circuits comprising at least two circuit connectors,
d. At least two stand-alone venturis or gas blenders,
e. At least two stand-alone combination valves that incorporate an inspiratory pressure relief valve, a time cycled pressure release valve, and an expiratory pressure relief valve,
f. At least one stand-alone manifold pressure regulator valve,
g. At least two stand-alone time controllers,
   wherein the system manifold comprises a first system manifold configured to connect to the pressure-flow regulator with a first manifold first connector, the at least one of the ventilator circuits with a first manifold second connector, and a second system manifold with a first manifold third connector,
   the second system manifold is configured to connect to the first system manifold with a second manifold first connector, at least one of the ventilator circuits with a second manifold second connector, and the manifold pressure regulator valve with a second manifold third connector,
   the at least one ventilator circuit is configured to connect to the at least one venturi or gas blender with a first circuit connector and the at least one combination valve with a first circuit connector, and
   the at least one time controller is configured to control the combination valve.

It is another object of the present disclosure to provide a modular ventilator apparatus comprising the components of the above-mentioned kit.

These and other objects will be described in further detail below and in the appended drawings.

### Brief Descriptions of the Drawings

Fig. 1 is a schematic diagram of a ventilator apparatus according to a first embodiment.
Fig. 2 is a flow diagram of the ventilator apparatus of the first embodiment.
Fig. 3 is a schematic diagram of a ventilator apparatus according to a second embodiment.
Fig. 4 is a flow diagram of the ventilator apparatus of the second embodiment.
Fig. 5 is a schematic diagram of a ventilator apparatus according to a third embodiment.
Fig. 6 is a flow diagram of the ventilator apparatus of the third embodiment.
Fig. 7 is a schematic diagram of a ventilator system according to a fourth embodiment.
Fig. 8 is a schematic diagram of a ventilator system according to a fifth embodiment.
Fig. 9 is a schematic diagram of a ventilator system according to a sixth embodiment.
Fig. 10 is a schematic diagram of a valve according to a preferred embodiment.

### Detailed Description of the Preferred Embodiments

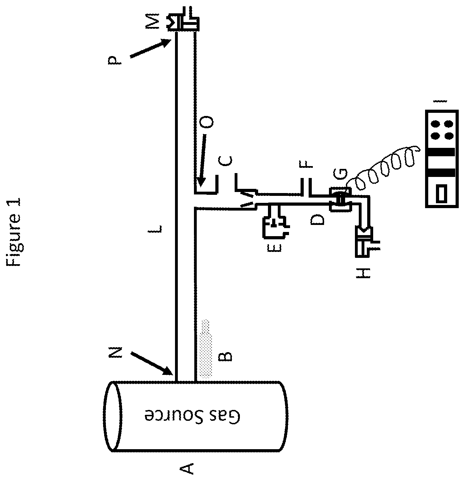

Fig. 1 is a schematic diagram of a ventilator apparatus according to a first embodiment. In Fig. 1, a gas source A provides pressurized gas through a flow/pressure regulator B to system manifold L via a connector N. The system manifold L is further connected to a ventilator circuit D via connector O. The ventilator circuit D comprises a venturi device C. The venturi device C delivers the pressurized gas to a patient (human or animal) or a (test or extracorporeal) lung via connector F. The ventilator circuit D further comprises an inspiratory pressure relief valve E, a time cycled pressure release valve G, and an expiratory pressure relief valve H. A time controller I controls time in the time cycled pressure release valve G. The system manifold is further connected to a manifold pressure regulator valve M at manifold connector P.

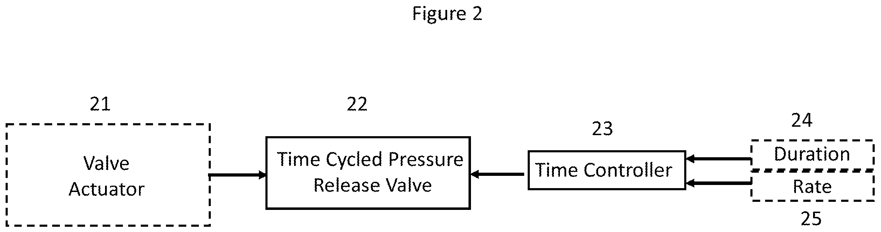

As seen in Fig. 1, the flow/pressure controller B regulates the flow of gas (i.e., oxygen) and pressure through the system manifold L and the ventilator circuit D from the gas source A. The gas passes from the gas source A through the system manifold L to the venturi valve C in the ventilator circuit D to entrain gas and increase the flow/pressure of gas through the ventilator circuit D and alter the gas concentration to the patient or lung at F as needed. The manifold pressure regulator valve M sets the threshold pressure above which pressure is released, which is the same pressure as set by the flow/pressure regulator B. The expiratory pressure relief valve G functions to maintain a resistance to gas flow to create pressure when the time cycled pressure release valve G is open. Fig. 2 is a flow diagram of the time cycled pressure release valve G of Fig. 1. As seen in Fig. 2, the time cycled pressure release valve 22 has a valve actuator 21 that is controlled by a time controller 23. The valve actuator 21 controls a restrictor (by depressurizing and repressurizing) to vary the gas flow in the ventilator circuit D. The time controller 23 adjusts the duration of time 24 that the valve actuator 21 is open and the rate 25 of how many times the valve actuator 21 opens and closes.

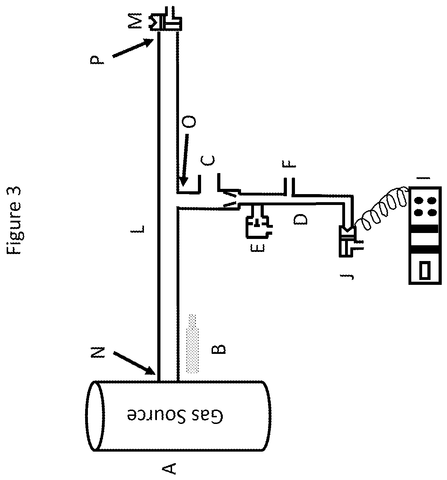

Fig. 3 is a schematic diagram of a ventilator apparatus according to a second embodiment, wherein the functionalities of the time cycled pressure release valve G and the expiratory pressure relief valve H have been combined into a combined pressure relief/timed threshold expiratory valve J. If the patient exhales or coughs, extra air moves through the inspiratory pressure relief valve E and escapes through valve J. Valve E is always open to some extent to allow flow through. Valve J is always closed unless the patient needs pressure relief or valve J opens on timed expiratory release controlled by time controller I.

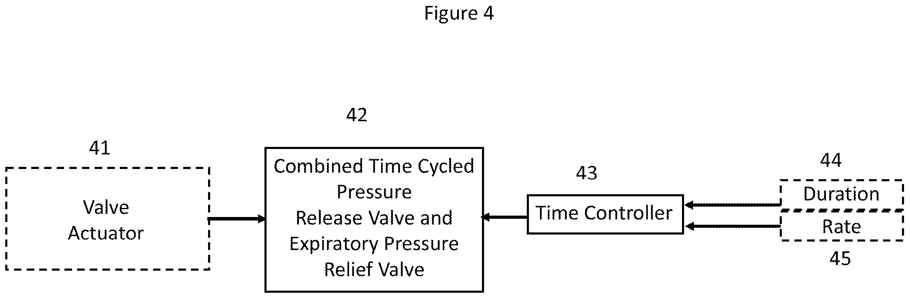

As seen in Fig. 3, the flow/pressure regulator B regulates the flow of gas (i.e. oxygen) and pressure through the system manifold L and the ventilator circuit D from the gas source A. The gas passes from the gas source A through the system manifold to the ventilator circuit D and the venturi valve C to entrain gas and increase the flow/pressure of gas through the circuit and alter the gas concentration to the patient connected at F. The inspiratory pressure relief valve E functions to maintain a resistance to gas flow to create pressure when the time threshold expiratory valve J is open. Fig. 4 is a flow diagram of the combined pressure relief/timed threshold expiratory valve J of Fig. 3. As seen in Fig. 4, a valve actuator 41 physically moves a restrictor to vary the gas flow of the combined time cycled pressure release valve and expiratory pressure relief valve 42 through a time controller 43 to adjust the duration of time 44 the valve actuator is open and the rate 45 of how many times the actuator 41 of the combined time cycled pressure release valve and expiratory pressure relief valve 42 opens and closes.

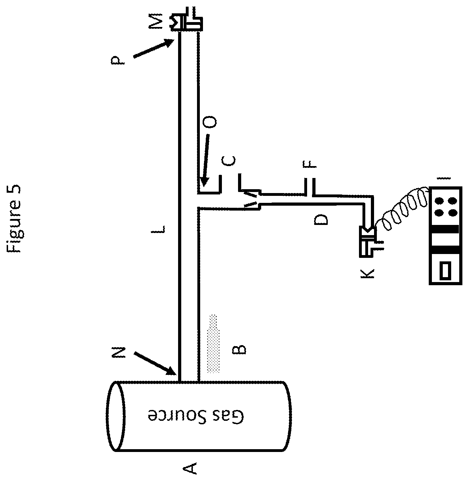

Fig. 5 is a schematic diagram a ventilator apparatus according to a third embodiment, wherein the inspiratory pressure relief valve E, the time cycled pressure release valve G, and the expiratory pressure relief valve H are combined into an inspiratory pressure relief valve/time cycled pressure release valve/expiratory pressure relief valve K. According to the third embodiment, the valve K provides pressure relief if the patient exhales/coughs, timed expiratory flow, and can also retard the flow to retain positive pressure in the expiratory limb.

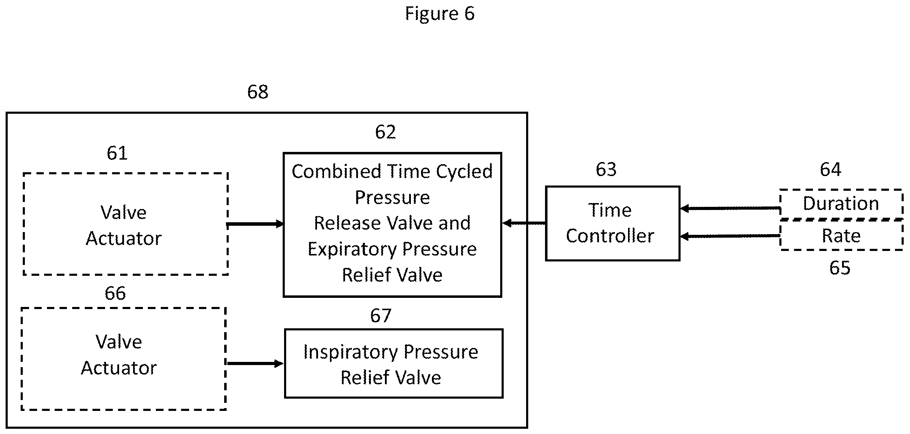

The flow/pressure controller B regulates the flow of gas (i.e., oxygen) and pressure through the system manifold L and the ventilation circuit D from a gas source A. The gas passes from the gas source A through the system manifold L, through the ventilation circuit D and the venturi valve C to entrain gas and increase the flow/pressure of gas through the circuit and alter the gas concentration to the patient connected at F. Fig. 6 is a flow diagram of the combined inspiratory pressure relief valve/time cycled pressure release valve/expiratory pressure relief valve K of Fig. 5. A seen in Fig. 6, the combined valve 68 comprises a valve actuator 61 that physically moves the restrictor to vary the gas flow of the combined time cycled pressure release valve/ expiratory pressure relief valve 62 and a valve actuator 66 physically moves the restrictor to vary the gas flow of the inspiratory pressure relief valve 67 where a time controller 63 adjusts the duration of time 64 the valve actuator is open and the rate 65 of how many times the actuator 61 of the combined time cycled pressure release valve/expiratory pressure relief valve 62 opens and/or closes.

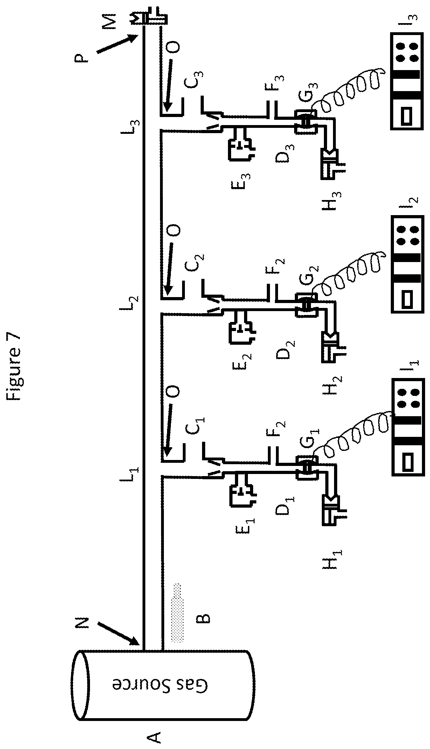

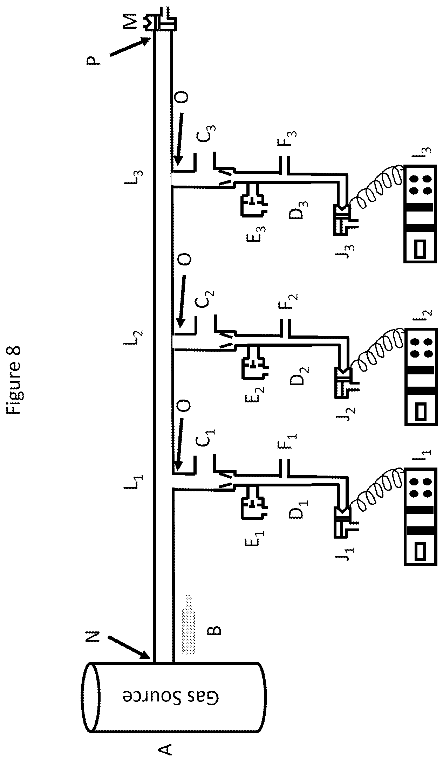

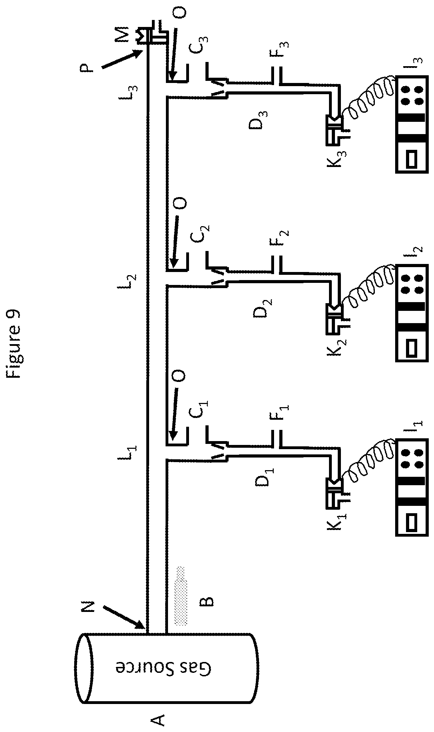

Fig. 7 is a schematic diagram of a ventilator system according to the present technology. In Fig. 7, multiple ventilator apparatus as shown in Fig. 1 are connected in series to one gas source via a system manifold L. In Fig. 8, multiple ventilator apparatus as shown in Fig. 3 are connected in series to one gas source via a system manifold L. In Fig. 9, multiple ventilator apparatus as shown in Fig. 5 are connected in series to one gas source via a system manifold L. Alternatively, the ventilator system could connect the apparatus of Figs. 1, 3 and 4 in any combination via a system manifold.

According to the present technology, the pressurized gas source A may be a wall source of gas at a pressure of about 20-60 psi (about 0,14-0,41 MPa), preferably about 40-50 psi (about 0,28-0,34 MPa), with flow that can be regulated by a flow/pressure regulator B. Alternatively, the pressurized gas source may be compressed gas, flow generator, turbine, or displacement pump. According to a preferred embodiment, the pressurized gas source is an axial flow turbine (vertical or centrifugal). A main line delivers pressurized gas through a gas tube to a patient and controls depressurization of the ventilator apparatus circuit. Oxygen can be delivered through a standard oxygen tank or a wall source. The oxygen concentration of the pressurized gas delivered to the patient depends on the ratio of the oxygen/air mix. A venturi device functions to accelerate and entrain the gas.

The patient may be connected to the ventilation apparatus by invasive or non-invasive means, including nasal cannula, mask, endotracheal tube, or tracheotomy.

The inspiratory pressure relief valve E may be a pop-up valve that is designed to relieve pressure if the patient exhales and the pressure in the expiratory limb exceeds a desired pressure. Preferably, the inspiratory pressure relief valve E maintains the pressure set by flow/pressure regulator B in the expiratory limb. The inspiratory pressure relief valve E is in open communication to the expiratory pressure relief valve H.

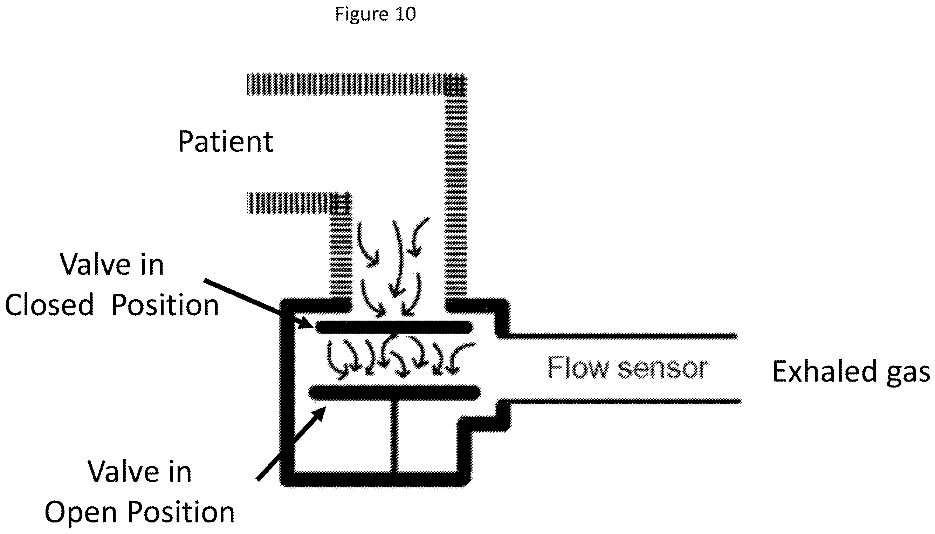

Fig. 10 is an example of a valve that may be used according to the present technology. The valve in Fig. 10 may have the following possible valve positions:
1. During the pressurization phase when delivering gas to the patient, the valve is in the closed position.
2. During the pressurization phase when delivering gas to the patient, the valve opens as a result of an expiratory effort of the patient and functions as a pressure relief valve. The degree to which the valve opens is based on the amount of pressure produced and dictates the position of the valve to be partially or fully open.
3. The time function of the valve may fully open the valve to depressurize to atmospheric pressure for a duration of time set at the time controller.
4. The time function of the valve may open to a varying degree to increase resistance thereby maintaining a pressure above atmospheric pressure for a duration of time set at the time controller.
5. The frequency of valve opening is controlled by the time controller.

The time controller I controls the expiratory time by controlling the time of gas that flows through the expiratory pressure relief valve H and the time cycled pressure release valve G. The time controller I also controls the pressure by controlling the aperture of the expiratory pressure relief valve H. Pressure is maintained above ambient by not opening the expiratory pressure relief valve H all the way. The expiratory pressure relief valve H controls both the time and the amount of gas flow. The expiratory pressure relief valve H can be a controlled by a time controller I or a manual valve (not shown).

The time control I may be any type of device that allows for repeated execution of a rule after an interval.

Each component of the ventilator apparatus is modular and can be easily assembled. The present technology contemplates a kit with various components for assembly of a ventilator apparatus and optionally, a ventilator system. Each component can be independently manufactured, such as by 3D printing, or other suitable method. As each component of the ventilator apparatus and system is modular, each component can be mixed/matched to suit the needs of multiple patients.

The ventilator apparatus and system may be used for time controlled adaptive ventilation mode that controls both pressure and time. The ventilator parts are modular, disposable and can be easily assembled without needing any special skill.

Preferably, the ventilator system monitors pressure and flow, and the volume is derived from calculating flow/time.

According to a preferred embodiment, the ventilator system includes a user interface that is removable and connectable wirelessly such as to create distance from the patient and a contaminated area. The user interface may be a touch screen that may be controlled by a mobile application. More preferably, the ventilator system may be remotely monitored from a central location.

## Claims

1. A modular ventilation system to ventilate at least one patient or lung, wherein the system comprises:
a. A port for connecting a single gas source (A) to the system to create a gas flow,
b. A pressure-flow regulator (B) to regulate a pressure in the gas flow,
c. At least one of a venturi device (C; C1) or gas blender to introduce oxygen into the gas flow,
d. A first system manifold (L; L1) to provide gas flow to the at least one patient or lung,
e. At least three pressure relief valves (E, H, M; E1, H1, M),
f. A time cycled pressure release valve (G; G1) to vary the gas flow in a ventilator circuit (D; D1) that is configured to deliver the gas flow to the at least one patient or lung, and
g. A time controller (I; I1) to control the time cycled pressure release valve (G; G1), Wherein the at least three pressure relief valves comprise an inspiratory pressure relief valve (E; E1) to regulate inspiratory patient pressure, an expiratory pressure relief valve (H; H1) to regulate expiratory patient pressure, and a manifold pressure regulator valve (M) to regulate manifold pressure,
wherein the first system manifold (L; L1) comprises at least three connectors, comprising a source connector (N) to connect the first system manifold to the gas source (A), a patient connector (O) to connect the first system manifold to the ventilator circuit (D; D1), and a manifold connector (P) to connect the first system manifold (L; L1) to the manifold pressure regulator valve (M) directly or indirectly via a second system manifold (L2), and
wherein the inspiratory pressure relief valve (E; E1), the time cycled pressure release valve (G; G1) and the expiratory pressure relief valve (H; H1) are proximal to the patient or the lung, and individually adjustable for each patient or lung.

2. The system of claim 1, wherein the gas source (A) is a turbine, compressed gas, or a flow generator.

3. The system of claim 1, wherein the venturi device (C; C1) is configured to introduce oxygen to produce a gas flow with less than 100% oxygen, preferably about 70% oxygen, or wherein the gas bender is configured to introduce oxygen to produce a gas flow that is up to 100% oxygen.

4. The system of claim 1, wherein the pressure-flow regulator (B) is configured to regulate a pressure from the gas source (A) to 0,14-0,41 MPa (20-60 psi), preferably about 0,28-0,34 MPa (about 40-50 psi).

5. The system of claim 1, wherein the inspiratory pressure relief valve (E; E1) is configured such that the inspiratory patient pressure is no more than 100 cm H₂0.

6. The system of claim 1, wherein the manifold pressure regulator valve (M) is configured such that the manifold pressure is 100 cm H₂O times a number of patients or lungs connected to the system.

7. The system of claim 1, further comprising an alarm that is configured to sound when a pressure threshold is not met.

8. The system of claim 1, wherein the at least three connectors are mechanical connections comprising threaded, snap, friction or quick connect connectors.

9. The system of claim 1, wherein the at least three pressure relief valves are mechanical valves comprising spring loaded pressure valves or pneumatic valves, or electrical valves comprising solenoid valves.

10. The system of claim 1, further comprising a power source, wherein the power source comprises a compressed gas driven turbine or electrical connection.

11. The system of claim 1, wherein the time cycled pressure release valve and the expiratory pressure relief valve are combined into one valve (J; J1), or wherein the inspiratory pressure relief valve, the time cycled pressure release valve, and the expiratory pressure relief valve are combined into one valve (K; K1).

12. The system of claim 1, wherein the system is configured to ventilate a patient or a lung with continuous positive pressure (CPAP) or cyclic ventilation by adjusting the inspiratory pressure relief valve and the expiratory pressure relief valve, or wherein the system is configured to ventilate a patient or a lung in a pressure and time-controlled mode by adjusting the inspiratory pressure relief valve and the time cycled pressure release valve.

13. A method of ventilating at least one lung with the modular ventilation system of claim 1, wherein the at least one lung is an extracorporeal lung or a test lung.

14. A kit for a modular ventilation system comprising:
a. At least two stand-alone system manifolds (L1, L2, L3) each comprising three manifold connectors,
b. A stand-alone pressure-flow regulator (B),
c. At least two stand-alone ventilator circuits (D1, D2, D3) each comprising at least two circuit connectors,
d. At least two stand-alone venturi devices (C1, C2, C3) or gas blenders,
e. At least two stand-alone combination valves (K1, K2, K3), each stand-alone combination valve (K1, K2, K3) incorporating an inspiratory pressure relief valve, a time cycled pressure release valve, and an expiratory pressure relief valve,
f. At least one stand-alone manifold pressure regulator valve (M),
g. At least two stand-alone time controllers (I1, I2, I3),
wherein the at least two stand-alone system manifolds comprise a first system manifold (L1) and a second system manifold (L2), wherein the first system manifold is configured to connect to the pressure-flow regulator (B) with a first manifold first connector (N), to at least one of the ventilator circuits (D1) with a first manifold second connector (O), and to the second system manifold (L2) with a first manifold third connector,
wherein the second system manifold (L2) is configured to connect to the first system manifold (L1) with a second manifold first connector, to at least one of the ventilator circuits (D2) with a second manifold second connector (O), and to the manifold pressure regulator valve (M) with a second manifold third connector (P), wherein each ventilator circuit is configured to connect to a respective venturi device or gas blender with a first circuit connector and to a respective combination valve with a second circuit connector, and
wherein each time controller is configured to control a respective combination valve.

15. A modular ventilator apparatus comprising the components of the kit of claim 14.

## Patentansprüche

1. Ein modulares Beatmungssystem zur Beatmung mindestens eines Patienten oder einer Lunge, wobei das System umfasst:
a. Ein Anschluss zum Anschluss einer einzelnen Gasquelle (A) an das System, um einen Gasfluss zu erzeugen,
b. Ein Druck-Durchflussregler (B) zur Regulierung des Drucks im Gasstrom,
c. Mindestens eine Venturi-Vorrichtung (C; C1) oder ein Gasmischer zur Einbringung von Sauerstoff in den Gasstrom,
d. Ein erster Systemverteiler (L; L1) zur Bereitstellung des Gasflusses zu mindestens einem Patienten oder einer Lunge,
e. Mindestens drei Druckbegrenzungsventile (E, H, M; E1, H1, M),
f. Ein zeitgesteuertes Druckablassventil (G; G1) zur Variation des Gasflusses in einem Beatmungskreislauf (D; D1), der so konfiguriert ist, dass er den Gasfluss an mindestens einen Patienten oder eine Lunge abgibt, und
g. Ein Zeitregler LI; L1) zur Steuerung des zeitgesteuerten Druckentlastungsventils (G; G1),
wobei die mindestens drei Druckbegrenzungsventile ein Inspirationsdruckbegrenzungsventil (E; E1) zur Regulierung des Inspirationsdrucks des Patienten, ein Exspirationsdruckbegrenzungsventil (H; H1) zur Regulierung des Exspirationsdrucks des Patienten und ein Verteilerdruckregelventil (M) zur Regulierung des Verteilerdrucks umfassen,
wobei der erste Systemverteiler (L; L1) mindestens drei Anschlüsse umfasst, einen Quellenanschluss (N) zum Verbinden des ersten Verteilersystems mit der Gasquelle (A), einen Patientenanschluss (O) zum Verbinden des ersten Systemverteilers mit dem Beatmungskreislauf (D; D1) und einen Verteileranschluss (P) zum Verbinden des ersten Systemverteilers (L; L1) mit dem Verteilerdruckregelventil (M) direkt oder indirekt über einen zweiten Systemverteiler (L2), und
wobei das Inspirationsdruckbegrenzungsventil (E; E1), das zeitgesteuerte Druckablassventil (G; G1) und das Exspirationsdruckbegrenzungsventil (H; H1) proximal zum Patienten bzw. zur Lunge angeordnet und für jeden Patienten bzw. jede Lunge individuell einstellbar sind.

2. Das System nach Anspruch 1, wobei die Gasquelle eine Turbine, Druckgas oder ein Strömungsgenerator ist.

3. Das System nach Anspruch 1, wobei die Venturi-Vorrichtung (C; C1) so konfiguriert ist, dass sie Sauerstoff einführt, um einen Gasstrom mit weniger als 100 % Sauerstoff, vorzugsweise etwa 70 % Sauerstoff, zu erzeugen, oder wobei der Gasmischer so konfiguriert ist, dass er Sauerstoff einführt, um einen Gasstrom zu erzeugen, der bis zu 100 % Sauerstoff enthält.

4. Das System nach Anspruch 1, wobei der Druck-Durchflussregler (B) so konfiguriert ist, dass er einen Druck von der Gasquelle (A) auf 0,14-0,41 MPa (20-60 psi), vorzugsweise etwa 0,28-0,34 MPa (etwa 40-50 psi) regelt.

5. Das System nach Anspruch 1, wobei das Inspirationsdruckbegrenzungsventil (E; E1) so konfiguriert ist, dass der Inspirationsdruck des Patienten nicht mehr als 100 cm H₂O beträgt.

6. Das System nach Anspruch 1, wobei das Verteilerdruckregelventil (M) so konfiguriert ist, dass der Verteilerdruck 100 cm H₂O mal der Anzahl der an das System angeschlossenen Patienten oder Lungen beträgt.

7. Das System nach Anspruch 1, das ferner einen Alarm umfasst, der so konfiguriert ist, dass er ertönt, wenn ein Druckschwellenwert nicht erreicht wird.

8. Das System nach Anspruch 1, wobei die mindestens drei Anschlüsse mechanische Verbindungen sind, die Gewinde-, Schnapp-, Reibungs- oder Schnellverbindungsverbindungen umfassen.

9. Das System nach Anspruch 1, wobei die mindestens drei Druckbegrenzungsventile mechanische Ventile, darunter federbelastete Druckventile, oder pneumatische Ventile, oder elektrische Ventile, darunter Magnetventile, sind.

10. Das System nach Anspruch 1, das ferner eine Energiequelle umfasst, wobei die Energiequelle eine mit Druckgas angetriebene Turbine oder eine elektrische Verbindung umfasst.

11. Das System nach Anspruch 1, wobei das zeitgesteuerte Druckablassventil und das Exspirationsdruckbegrenzungsventil zu einem Ventil (J; J1) kombiniert sind, oder wobei das Inspirationsdruckbegrenzungsventil, das zeitgesteuerte Druckablassventil und das Exspirationsdruckbegrenzungsventil zu einem Ventil (K; K1) kombiniert sind.

12. Das System nach Anspruch 1, wobei das System dazu konfiguriert ist, einen Patienten oder eine Lunge mit kontinuierlichem positivem Druck (CPAP) oder zyklischer Beatmung zu beatmen, indem das Inspirationsdruckbegrenzungsventil und das Exspirationsdruckebegrenzungsventil eingestellt werden, oder wobei das System dazu konfiguriert ist, einen Patienten oder eine Lunge in einem druck- und zeitgesteuerten Modus zu beatmen, indem das Inspirationsdruckbegrenzungsventil und das zeitgesteuerte Druckablassventil eingestellt werden.

13. Verfahren zur Beatmung mindestens einer Lunge mit dem modularen Beatmungssystem nach Anspruch 1, wobei die mindestens eine Lunge eine extrakorporale Lunge oder eine Testlunge ist.

14. Bausatz für ein modulares Lüftungssystem, umfassend:
a. Mindestens zwei eigenständige Systemverteiler (L1, L2, L3) mit jeweils drei Verteileranschlüssen,
b. Ein eigenständiger Druck-Durchflussregler (B),
c. Mindestens zwei eigenständige Beatmungskreisläufe (D1, D2, D3) mit jeweils mindestens zwei Kreisanschlüssen,
d. Mindestens zwei eigenständige Venturi-Vorrichtungen (C1, C2, C3) oder Gasmischer,
e. Mindestens zwei eigenständige Kombinationsventile (K1, K2, K3), wobei jedes eigenständige Kombinationsventil (K1, K2, K3) ein Inspirationsdruckbegrenzungsventil, ein zeitgesteuertes Druckablassventil und ein Exspirationsdruckbegrenzungsventil enthält,
f. Mindestens ein eigenständiges Verteilerdruckregelventil (M),
g. Mindestens zwei eigenständige Zeitregler (I1, I2, I3),
wobei die mindestens zwei eigenständigen Systemverteiler einen ersten Systemverteiler (L1) und einen zweiten Systemverteiler (L2) umfassen, wobei der erste Systemverteiler so konfiguriert ist, dass er mit einem ersten Verteileranschluss (N) an den Druck-Durchflussregler (B), mit einem zweiten Verteileranschluss (O) an mindestens einen der Beatmungskreisläufe (D1) und mit einem dritten Verteileranschluss an den zweiten Systemverteiler (L2) angeschlossen werden kann,
wobei der zweite Systemverteiler (L2) so konfiguriert ist, dass er mit einem ersten Anschluss des zweiten Verteilers an den ersten Systemverteiler (L1), mit einem zweiten Anschluss (O) des zweiten Verteilers an mindestens einen der Beatmungskreisläufe (D2) und mit einem dritten Anschluss (P) des zweiten Verteilers an das Verteilerdruckregelventil (M) angeschlossen werden kann, wobei jeder Beatmungskreis so konfiguriert ist, dass er über einen ersten Kreisanschluss mit einem entsprechenden Venturi-Vorrichtung oder Gasmischer und über einen zweiten Kreisanschluss mit einem entsprechenden Kombinationsventil verbunden werden kann, und
wobei jeder Zeitregler dazu konfiguriert ist, ein jeweiliges Kombinationsventil zu steuern.

15. Ein modulares Beatmungsgerät, das die Komponenten des Kits nach Anspruch 14 umfasst.

## Revendications

1. Un système de ventilation modulaire pour ventiler au moins un patient ou un poumon, dans lequel le système comprend:
a. Un port permettant de connecter une seule source de gaz (A) au système pour créer un flux de gaz,
b. Un régulateur de pression-débit (B) pour réguler la pression dans le flux de gaz,
c. Au moins un dispositif Venturi (C ; C1) ou un mélangeur de gaz pour introduire de l'oxygène dans le flux de gaz,
d. Un premier collecteur de système (L ; L1) pour fournir un flux de gaz à au moins un patient ou poumon,
e. Au moins trois soupapes de surpression (E, H, M ; E1, H1, M),
f. Une soupape de décharge de pression à cycle temporel (G ; G1) permettant de faire varier le débit de gaz dans un circuit de ventilation (D ; D1) configuré pour délivrer le débit de gaz à au moins un patient ou poumon, et
g. Un contrôleur de temps (L; L1) pour contrôler la soupape de décharge de pression
à cycle temporel (G ; G1)
dans lequel les au moins trois soupapes de surpression comprennent une soupape de surpression inspiratoire (E ; E1) pour réguler la pression inspiratoire du patient, une soupape de surpression expiratoire (H ; H1) pour réguler la pression expiratoire du patient et une soupape de régulation de pression du collecteur (M) pour réguler la pression du collecteur,
dans lequel le premier collecteur de système (L ; L1) comprend au moins trois connecteurs, comprenant un connecteur de source (N) pour connecter le premier collecteur de système à la source de gaz (A), un connecteur patient (O) pour connecter le premier collecteur de système au circuit du ventilation (D ; D1), et un connecteur de collecteur (P) pour connecter le premier collecteur de système (L ; L1) à la soupape de régulation de pression du collecteur (M) directement ou indirectement via un second collecteur de système (L2), et
dans laquelle la soupape de surpression inspiratoire (E ; E1), la soupape de décharge de pression à cycle temporel (G ; G1) et la soupape de surpression expiratoire (H ; H1) sont proches du patient ou du poumon et réglables individuellement pour chaque patient ou poumon.

2. Système selon la revendication 1, dans lequel la source de gaz est une turbine, un gaz comprimé ou un générateur de flux.

3. Système selon la revendication 1, dans lequel le dispositif Venturi (C ; C1) est configuré pour introduire de l'oxygène afin de produire un flux de gaz contenant moins de 100 % d'oxygène, de préférence environ 70 % d'oxygène, ou dans lequel le mélangeur de gaz est configuré pour introduire de l'oxygène afin de produire un flux de gaz contenant jusqu'à 100 % d'oxygène.

4. Système selon la revendication 1, dans lequel le régulateur de pression-débit (B) est configuré pour réguler une pression provenant de la source de gaz (A) à 0,14-0,41 Mpa (20-60 psi), de préférence environ 0,28-0,34 Mpa (environ 40-50 psi).

5. Système selon la revendication 1, dans lequel la soupape de surpression inspiratoire (E ; E1) est configurée de telle sorte que la pression inspiratoire du patient ne soit pas supérieure à 100 cm H₂0.

6. Système selon la revendication 1, dans lequel la soupape de régulation de pression du collecteur (M) est configurée de telle sorte que la pression du collecteur est de 100 cm H₂O multipliée par un nombre de patients ou de poumons connectés au système.

7. Système selon la revendication 1, comprenant en outre une alarme qui est configurée pour retentir lorsqu'un seuil de pression n'est pas atteint.

8. Système selon la revendication 1, dans lequel les au moins trois connecteurs sont des connexions mécaniques comprenant des connecteurs filetés, à encliquetage, à friction ou à connexion rapide.

9. Système selon la revendication 1, dans lequel les au moins trois soupapes de surpression sont des soupapes mécaniques comprenant des soupapes de pression à ressort ou des soupapes pneumatiques, ou des soupapes électriques comprenant des électrovannes.

10. Système selon la revendication 1, comprenant en outre une source d'alimentation, la source d'alimentation comprenant une turbine à gaz comprimé ou une connexion électrique.

11. Système selon la revendication 1, dans lequel la soupape de décharge de pression à cycle temporel et la soupape de surpression expiratoire sont combinées en une seule soupape (J ; J1), ou dans lequel la soupape de surpression inspiratoire, la soupape de décharge de pression à cycle temporel et la soupape de surpression expiratoire sont combinées en une seule soupape (K ; K1).

12. Système selon la revendication 1, dans lequel le système est configuré pour ventiler un patient ou un poumon avec une pression positive continue (CPAP) ou une ventilation cyclique en ajustant la soupape de surpression inspiratoire et la soupape de surpression expiratoire, ou dans lequel le système est configuré pour ventiler un patient ou un poumon dans un mode contrôlé par pression et temps en ajustant la soupape de surpression inspiratoire et la soupape de décharge de pression à cycle temporel.

13. Procédé de ventilation d'au moins un poumon avec le système de ventilation modulaire selon la revendication 1, dans lequel le ou les poumons sont un poumon extracorporel ou un poumon d'essai.

14. Un kit pour un système de ventilation modulaire comprenant:
a. Au moins deux collecteurs de système autonomes (L1, L2, L3) comprenant chacun trois connecteurs de collecteur,
b. Un régulateur de pression-débit autonome (B),
c. Au moins deux circuits de ventilation autonomes (D1, D2, D3) comprenant chacun au moins deux connecteurs de circuit,
d. Au moins deux dispositifs Venturi autonomes (C1, C2, C3) ou mélangeurs de gaz,
e. Au moins deux valves combinées autonomes (K1, K2, K3), chaque valve combinée autonome (K1, K2, K3) incorporant une valve de surpression inspiratoire, une valve de décharge de pression à cycle temporel et une valve de surpression expiratoire,
f. Au moins une soupape de régulation de pression de collecteur autonome (M),
g. Au moins deux contrôleurs de temps autonomes (I1, I2, I3),
dans lequel les au moins deux collecteurs de système autonomes comprennent un premier collecteur de système (L1) et un second collecteur de système (L2), dans lequel le premier collecteur de système est configuré pour se connecter au régulateur de pression-débit (B) avec un premier connecteur de collecteur (N), à au moins l'un des circuits de ventilation (D1) avec un premier connecteur de collecteur (O), et au second collecteur de système (L2) avec un premier connecteur de collecteur (3),
dans lequel le deuxième collecteur de système (L2) est configuré pour se connecter au premier collecteur de système (L1) avec un deuxième premier connecteur de collecteur, à au moins l'un des circuits de ventilation (D2) avec un deuxième deuxième connecteur de collecteur (O), et à la soupape de régulation de pression du collecteur (M) avec un deuxième troisième connecteur de collecteur (P),
dans lequel chaque circuit de ventilation est configuré pour se connecter à un dispositif Venturi ou à un mélangeur de gaz respectif avec un premier connecteur de circuit et à une soupape combinée respective avec un second connecteur de circuit, et
dans lequel chaque contrôleur de temps est configuré pour contrôler une soupape combinée respective.

15. Appareil de ventilation modulaire comprenant les composants du kit selon la revendication 14.
